# EUROPÄISCHE PATENTSCHRIFT

(11) **EP 0 524 996 B1**
(45) Veröffentlichungstag und Bekanntmachung des Hinweises auf die Patenterteilung: **28.06.1995**
(21) Anmeldenummer: 91907450.0
(22) Anmeldetag: 05.04.1991
(51) Int. Cl.: C07C 309/17, C07C 303/06, C07C 67/54, C07C 67/56

(54) **VERFAHREN ZUR HERSTELLUNG HELLFARBIGER WASCHAKTIVER ALPHA-SULFOFETTSÄURENIEDRIGALKYLESTER-SALZE**
PROCESS FOR MANUFACTURING LIGHT-COLOURED WASHING ALPHA-SULPHO-FATTY-ACID LOW-ALKYL ESTER SALTS
PROCEDE DE FABRICATION DE SELS D'ALKYLESTERS INFERIEURS D'ACIDES GRAS ALPHA-SULFONES DETERGENTS DE TEINTE CLAIRE

(30) Priorität: 14.04.1990 DE 4012106
(43) Veröffentlichungstag der Anmeldung: 03.02.1993
(73) Patentinhaber: Henkel Kommanditgesellschaft auf Aktien, 40191 Düsseldorf (DE)
(72) Erfinder: FABRY, Bernd, D-4052 Korschenbroich 1 (DE); KRATZEL, Ulrich, D-8650 Kulmbach (DE); SCHMIDT, Wolfgang, D-4019 Monheim (DE); KREIENFELD, Günter, D-4000 Düsseldorf (DE)
(86) Internationale Anmeldenummer: EP9100644
(87) Internationale Veröffentlichungsnummer: WO9116300

(56) Entgegenhaltungen:
- EP-A- 0 222 237

## Beschreibung

Gegenstand der Erfindung ist ein Verfahren zur Herstellung hellfarbiger waschaktiver alpha-Sulfofettsäureniedrigalkylester-Salze durch Vorbehandlung von Fettsäureniedrigalkylestern, Umsetzung mit gasförmigem Schwefeltrioxid und nachfolgende Aufarbeitung.

Die Sulfonierung von Fettsäureniedrigalkylestern zur Herstellung von alpha-Sulfofettsäureniedrigalkylester-Salzen, die nachfolgend als "Estersulfonate" bezeichnet werden, ist seit langem bekannt **[J.Am.Oil.Chem.Soc. 39, 490 (1962)]**. Als technisches Ausgangsmaterial werden Fette und/oder Öle pflanzlichen oder tierischen Ursprungs verwendet, aus denen die Fettsäureniedrigalkylester entweder durch Fettspaltung und nachfolgende Veresterung mit niederen Alkoholen oder durch Umesterung der natürlichen Triglyceride mit niederen Alkoholen hergestellt werden. Die resultierenden Fettsäureniedrigalkylester stellen Gemische dar, die - je nach Ursprung des natürlichen Rohstoffs - Ester von Fettsäuren mit 6 bis 22 Kohlenstoffatomen enthalten. Technische Fettsäureniedrigalkylester dieser Art werden nach bekannten Verfahren, die beispielsweise in den deutschen Patentanmeldungen **DE-A-12 48 645** und **DE-A-11 79 931** beschrieben sind, mit gasförmigem Schwefeltrioxid sulfoniert. Dies führt zu mehr oder minder stark verfärbten, sauren Rohprodukten, die gebleicht und durch Neutralisation bei pH 6 bis 7 in die entsprechenden alpha-Sulfofettsäureniedrigalkylester-Salze überführt werden. In dieser Form haben die Estersulfonate große Bedeutung als oberflächenaktive Substanzen für die Herstellung von Wasch- und Reinigungsmitteln.

Trotz optimaler Sulfonierungsbedingungen werden bei der Herstellung von Estersulfonaten jedoch häufig dunkelfarbige Produkte erhalten, deren Farbe unter Einsatz üblicher Bleichverfahren nicht oder nicht ausreichend aufgehellt werden kann. Als kritisch werden dabei Produkte angesehen, deren Klettfarbzahl nach der Bleiche, bestimmt in einer 1 cm Rundküvette und bei einer Konzentration von 5 Gew.-% Waschaktivsubstanz in Wasser, oberhalb von 30 liegt. Estersulfonate dieser Art kommen aus aesthetischen Gründen für die Herstellung von Wasch- und Reinigungsmitteln nicht in Betracht.

In der Vergangenheit hat es nicht an Versuchen gemangelt, Verfahren zu entwickeln, die die Herstellung hellfarbiger Estersulfonate sicherstellen.

Gemäß der Lehre der deutschen Patentanmeldung **DE-A-12 46 718** ist es für die Herstellung hellfarbiger Estersulfonate unerläßlich, nur solche Fettsäureester in die Sulfonierungsstufe einzusetzen, deren Gehalt an ungesättigten Verbindungen 0,1 bis 0,5 Gew.-% nicht übersteigt. In der technischen Anwendung hat es sich jedoch gezeigt, daß auch Fettsäureniedrigalkylester mit einem Gehalt an ungesättigten Verbindungen von 0,1 bis 0,3 Gew.-% zu Estersulfonaten mit unbefriedigender Farbqualität Anlaß geben können.

In der deutschen Patentschrift **DE-C-12 62 265** wird zur Verbesserung der Farbqualität vorgeschlagen, Mischungen von Fettsäureniedrigalkylestern und mindestens 25 Gew.-% eines Alkylbenzols einer gemeinsamen Umsetzung mit Schwefeltrioxid zu unterwerfen. Auf diesem Wege sind jedoch nur Aniontensidgemische zugänglich, die neben Estersulfonaten auch Alkylbenzolsulfonate enthalten.

Aus der deutschen Patentanmeldung **DE-A-14 43 995** geht hervor, daß sich Estersulfonate mit guter Farbqualität erhalten lassen, wenn man den Umsetzungsgrad während der Sulfonierung auf etwa 90 % begrenzt. Estersulfonate mit niedrigem Sulfonierungsgrad führen jedoch zu Problemen bei der Herstellung von Pulverwaschmitteln durch Zerstäuben, da die Verarbeitung solcher Produkte erfahrungsgemäß mit einem hohen Pluming verbunden ist.

Aus der europäischen Patentschrift **EP-B-0 054 724** ist schließlich ein Verfahren zur Herstellung hellfarbiger Estersulfonate bekannt, nach dem man Fettsäureester in die Sulfonierungsstufe einsetzt, deren Gehalt an Fettsäureglyceriden zuvor durch Destillation auf Werte von 0,3 bis 0,5 Gew.-% vermindert wurde. In der technischen Anwendung hat sich jedoch gezeigt, daß auch diese Maßnahme allein nicht ausreichend ist, um die Herstellung hellfarbiger Estersulfonate unabhängig vom Rohstoff und seiner Vorbehandlung sicherzustellen.

Die Aufgabe der Erfindung bestand somit darin, ein verbessertes Verfahren zur Herstellung hellfarbiger waschaktiver alpha-Sulfofettsäureniedrigalkylester-Salze zu entwickeln, das frei von den geschilderten Nachteilen ist.

Gegenstand der Erfindung ist ein Verfahren zur Herstellung hellfarbiger waschaktiver alpha-Sulfettsäureniedrigalkylester-Salze durch Sulfonierung von Fettsäureniedrigalkylestern mit gasförmigem Schwefeltrioxid und nachfolgende Aufarbeitung, dadurch gekennzeichnet, daß man in die Sulfonierungsstufe Fettsäureniedrigalkylester einbringt,
a) deren Gehalt an Oxoverbindungen auf Werte kleiner 0,1 Gew.-% und
b) deren Gehalt an ungesättigten Verbindungen auf Werte kleiner 0,1 Gew.-%,
Gewichtsprozente jeweils bezogen auf Fettsäureniedrigalkylester, zuvor vermindert worden ist.

Die Erfindung beruht auf der Erkenntnis, daß für die Bildung dunkelfarbiger Estersulfonate in erster Linie der Gehalt an Oxoverbindungen und ungesättigten Verbindungen im Fettsäureniedrigalkylester entscheidend ist. Die Erfindung schließt ferner die Erkenntnis ein, daß sich die genannten Stoffe in ihrer farbverschlechternden Wirkung gegenseitig beeinflußen und daß nur Maßnahmen, die auf die Verminderung der Anteile beider Gruppen von Farbverursachern zielen, geeignet sind, die Herstellung hellfarbiger Estersulfonate sicherzustellen.

Zur Herstellung hellfarbiger Estersulfonate werden in die Sulfonierungsstufe Fettsäureniedrigalkylester eingesetzt, deren Gehalt an Oxoverbindungen zuvor durch
a1) Destillation in Gegenwart von anorganischen Säuren oder deren Partialester mit Glycerin und/oder
a2) Adsorption an anorganischen Feststoffen
auf Werte kleiner 0,1, vorzugsweise kleiner 0,05 Gew.-%, bezogen auf den Fettsäureniedrigalkylester, vermindert wurde.

Als störende Oxoverbindungen kommen insbesondere Hydroxy-, Dihydroxy- und Ketofettsäureester in Betracht, die durch Autoxidation ungesättigter Fettsäureester als Verunreinigungen in Fettsäureniedrigalkylestern enthalten sind. Zur Gruppe der Oxoverbindungen sind ferner Sterinderivate, wie beispielsweise Cholesterin oder Cholesterol zu zählen, die Begleitstoffe natürlicher Fettsäureester insbesondere tierischer Herkunft darstellen.

Die anorganischen Säuren werden in einer Konzentration von 0,05 bis 0,5 Gew.-%, bezogen auf den Fettsäureniedrigalkylester, in die Destillationsstufe eingesetzt. Als anorganische Säuren kommen dabei Phosphorsäure, Unterphosphorige Säure und insbesondere Borsäure sowie deren Partialester mit Glycerin in Betracht. Die Destillation kann kontinuierlich oder diskontinuierlich bei einer Temperatur von 230 bis 280°C und einem Druck von 0,05 bis 0,2 mbar durchgeführt werden. Vorzugsweise wird zunächst die anorganische Säure bei 30 bis 40°C in den Fettsäureniedrigalkylester eingetragen und die Mischung dann einer Über-Kopf-Destillation unterworfen, bei der die anorganische Säure mit den Oxoverbindungen schwerflüchtige Ester bildet, die zum überwiegenden Teil im Destillationssumpf verbleiben.

Die anorganischen Feststoffe werden in einer Konzentration von 10 bis 150, vorzugsweise 25 bis 100 Gew.-%, bezogen auf den Fettsäureniedrigalkylester, in die Adsorptionsstufe eingesetzt. Als anorganische Festoffe kommen dabei Aluminiumoxid, Titandioxid, Magnesiumoxid und insbesondere Silicagel mit einer Korngröße von 0,03 bis 0,3 mm, vorzugsweise 0,06 bis 0,2 mm, in Betracht. Die anorganischen Feststoffe werden dem Fettsäureniedrigalkylester zugesetzt, das Gemisch unter Rühren in einer Schutzgasatmossphäre über einen Zeitraum von 0,5 bis 5 h auf 40 bis 80°C, vorzugsweise 40 bis 70°C erhitzt und der anorganischen Feststoff anschließend auf mechanischem Wege, z. B. durch Filtration bei 40 bis 70°C, wieder abgetrennt. Die polaren Oxoverbindungen adsorbieren an den anorgaschen Feststoffen und verbleiben zum überwiegenden Teil im Filtrationsrückstand, der nach Aufarbeitung wieder in die Adsorptionsstufe zurückgeführt werden kann.

Zur Herstellung hellfarbiger Estersulfonate werden in die Sulfonierungsstufe desweiteren Fettsäureniedrigalkylester eingesetzt, deren Gehalt an ungesättigten Verbindungen zuvor durch Härtung in Gegenwart üblicher Hydrierkatalysatoren auf Werte kleiner 0,1, vorzugsweise kleiner 0,05 Gew.-%, bezogen auf den Fettsäureniedrigalkylester, vermindert wurde.

Als störende ungesättigte Verbindungen kommen insbesondere die Ester der niederen Alkohole mit ungesättigten Fettsäuren, sowie Fettsäureester des Cholesterins in Betracht. Die Härtung kann beispielsweise unter Einsatz von 0,5 bis 2, vorzugsweise 0,8 bis 1,2 Gew.-%, bezogen auf die Gesamtmenge Fettsäureester, eines handelsüblichen Nickelkatalysators bei Temperaturen von 150 bis 220, vorzugsweise 170 bis 190°C und 5 bis 50, vorzugsweise 15 bis 25 bar durchge- führt werden. Durch die Härtung werden ungesättigte Fettsäureester in gesättigte Fettsäureester überführt.

In die Sulfonierungsstufe werden Ester von Fettsäuren mit 6 bis 22 Kohlenstoffatomen mit Alkoholen mit 1 bis 4 Kohlenstoffatomen, vorzugsweise Methylester von Fettsäuren mit 8 bis 18 Kohlenstoffatomen eingesetzt.

Die Herstellung der Fettsäureester kann über die Spaltung von natürlichen Fetten und Ölen und nachfolgende Veresterung mit niederen Alkoholen oder den Weg der direkten Umesterung mit niederen Alkoholen erfolgen.

In einer bevorzugten Ausführungsform der Erfindung werden Fettsäureniedrigalkylester eingesetzt, die durch Spaltung natürlicher Fette und Öle, Reinigung der resultierenden Fettsäuren nach dem Umnetzverfahren und nachfolgende Veresterung mit Methanol hergestellt werden.

Bei der Reinigung nach dem Umnetzverfahren **[Fat.Sci.Technol., 89, 237 (1987)]** wird das nach der Spaltung erhaltene Gemisch gesättigter und ungesättigter Fettsäuren auf Temperaturen von 2 bis 7°C abgekühlt, wobei die gesättigten Fettsäuren kristallisieren und mit den flüssigen ungesättigten Fettsäuren eine Dispersion bilden. Durch Zugabe eines Netzmittels, beispielsweise einer wässrigen Lösung eines C_{16/18}-Alkylsulfates, werden die ungesättigten Fettsäuren emulgiert, wobei die Kristalle der gesättigten Fettsäuren von anhaftenden ungesättigten Fettsäuren gereinigt werden. Beim anschließenden Zentrifugieren dieser Emulsion/Dispersion erfolgt eine Auftrennung in eine Phase, die die ungesättigten Fettsäuren enthält, und eine Dispersion gesättigter Fettsäuren in Wasser, die in einem Separator voneinander getrennt werden. Die Dispersion wird anschließend auf 70°C erwärmt und die geschmolzenen gesättigten Fettsäuren von der Wasserphase abgetrennt, wobei das Wasser in den Trennprozeß zurückkehrt.

Das Umnetzverfahren ist geeignet, den Gehalt ungesättigter Verbindungen und bestimmter Oxoverbindungen herabzusetzen. Im Sinne der Erfindung führt eine Kombination der Verfahrensmerkmale
1. Umnetzung,
2. Destillation in Gegenwart anorganischer Säuren und/oder
3. Adsorption an anorganischen Feststoffen und
4. Härtung
in synergistischer Weise zu besonders hellfarbigen Estersulfonaten.

Für den Erfolg der erfindungsgemäßen Vorbehandlung ist die Reihenfolge der genannten Verfahrensschritte unerheblich. Um den Materialverlust während der Aufarbeitung zu begrenzen, ist es jedoch empfehlenswert, die angegebene Reihenfolge einzuhalten.

Die Sulfonierung der Fettsäureniedrigalkylester erfolgt nach den Verfahren des Standes der Technik unter Einsatz eines Gemisches aus gasförmigem Schwefeltrioxid und einem Inertgas, vorzugsweise Stickstoff oder Luft, das üblicherweise 2 bis 8 Vol.-% Schwefeltrioxid enthält. Das molare Verhältnis von Fettsäureniedrigalkylester zu Schwefeltrioxid beträgt 1 : 1,1 und 1 : 1,8, vorzugsweise 1 : 1,2 und 1 : 1,5. Die Umsetzung erfolgt in einem Fallfilm- oder Kaskadenreaktor bei 70 bis 90°C. Nach der Sulfonierung wird das erhaltene saure Reaktionsgemisch bei 70 bis 90°C über einen Zeitraum von 10 bis 30 min einer Nachreaktion (Alterung) unterworfen und anschließend gebleicht und neutralisiert.

Die Bleiche kann nach an sich bekannten Verfahren in wässrigem Medium mit Wasserstoffperoxid und/oder Hypochlorit erfolgen. Die Neutralisation des sauren Reaktionsproduktes kann dabei sowohl vor als auch nach der Bleiche durchgeführt werden. Eine Bleiche mit Wasserstoffperoxid vor der Neutralisation ist beispielsweise in der deutschen Patentschrift **DE-B-11 79 931** beschrieben. Die deutsche Patentanmeldung **DE-A-12 34 709** beansprucht eine Kombinationsbleiche, bei der sich an die Behandlung des sauren Reaktionsproduktes mit wässrigem Wasserstoffperoxid die Neutralisation des teilweise gebleichten Sulfonierungsproduktes anschließt, worauf erneut mit Wasserstoffperoxid oder Natriumhypochlorit ein abschließender Bleichprozeß durchgeführt wird. Nach der Lehre der deutschen Patentanmeldung **DE-A-33 19 591** wird das rohe Sulfonierungsprodukt zunächst in einem neutral bis schwach alkalisch eingestellten wässrigem Medium einer Vorbleiche mit Natriumhypochlorit unterworfen, ehe die Salzpaste schwach sauer eingestellt und mit Wasserstoffperoxid oder Peroxidverbindungen nachgebleicht wird.

Die Neutralisation der sauren Sulfonierungsprodukte wird mit wässrigen Alkali- oder Erdalkalihydroxidlösungen, vorzugsweise mit wässriger Natriumhydroxidlösung durchgeführt. Bei der Bleiche und der Neutralisation sind die Verfahrensbedingungen derart zu wählen, daß die hier prinzipiell mögliche Esterverseifung weitgehend ausgeschlossen wird.

Die nachfolgenden Beispiele sollen den Gegenstand der Erfindung näher erläutern ohne ihn darauf einzuschränken.

### Beispiele

### I. Ausgangsester

**Ester I.** Talgfettsäuremethylester gewonnen durch Druckspaltung von Rindertalg, weitgehende Abtrennung ungesättigter Fettsäuren nach dem Umnetzverfahren und Veresterung mit Methanol. Die Zusammensetzung sowie die fettchemischen Kennzahlen des Esters sind in Tab.1 zusammengefaßt.

**Ester II.** Talgfettsäuremethylester gewonnen durch Umesterung von Rindertalg mit Methanol. Die Zusammensetzung sowie die fettchemischen Kennzahlen des Esters sind in Tab.1 zusammengefaßt.

**Ester III.** Palmstearinfettsäuremethylester gewonnen durch Druckspaltung von Palmöl, weitgehende Abtrennung ungesättigter Fettsäuren nach dem Umnetzverfahren und Veresterung mit Methanol. Die Zusammensetzung sowie die fettchemischen Kennzahlen des Esters sind in Tab.1 zusammengefaßt.

**Tab.1**

| Zusammensetzung und fettchemische Kennzahlen der Ester Angaben zur Zusammensetzung in Gew.-% | | | |
|---|---|---|---|
| | Ester I | Ester II | Ester II |
| C₁₂-Fettsäureester | 2 | 1 | - |
| C₁₄-Fettsäureester | 5 | 3 | 4 |
| C₁₆-Fettsäureester | 53 | 27 | 79 |
| C₁₈-Fettsäureester | 40 | 69 | 17 |
| Oxoverbindungen | 0,12 | 0,25 | 0,11 |
| Iodzahl | 21 | 47 | 21 |
| Hydroxylzahl | 1,1 | 1,1 | 1,1 |
| Verseifungszahl | 195,8 | 192,5 | 198,3 |
| Säurezahl | 0,5 | 0,7 | 0,4 |

### II. Vorbehandlungsverfahren

Zur Vorbehandlung der Fettsäureniedrigalkylester wurden folgende Verfahrensschritte durchgeführt:
1. Destillation,
2. Destillation in Gegenwart von 0,1 Gew.-% Borsäure,
3. Adsorption unter Einsatz von 75 Gew.-% Silicagel (Kieselgel 60^{(R)}, Körnung 0,063 - 0,2 mm, Fa.Merck), Rührzeit 3h, Rührtemperatur 80°C
4. Härtung
Einzelheiten zu den erfindungsgemäßen Verfahren (A - D) sowie zu den Vergleichsverfahren (E - K) sind in Tab.2 zusammengefaßt.

**Tab.2**

| Vorbehandlungsverfahren | | | | | |
|---|---|---|---|---|---|
| Verfahren | 1. | 2. | 3. | 4. | Iodzahl |
| A | - | + | - | + | 0,09 |
| B | - | + | - | + | 0,05 |
| C | - | - | + | + | 0,05 |
| D | - | + | + | + | 0,05 |
| E | + | - | - | + | 0,50 |
| F | + | - | - | + | 0,10 |
| G | + | - | - | + | 0,05 |
| H | - | + | - | + | 0,50 |
| I | - | + | - | + | 0,30 |
| J | - | - | + | + | 0,50 |
| K | - | - | + | + | 0,30 |

### III. Sulfonierung und Aufarbeitung

Die Sulfonierung der Fettsäureniedrigalkylester und die Aufarbeitung des sauren Sulfonierungsproduktes erfolgte nach einem Standardverfahren:

In einem 1-l-Sulfierreaktor mit Heiz- und Kühlmantel und Gaseinleitungsrohr wurde 1 Mol Fettsäureester auf 80°C erwärmt und mit 9,6 g (1,2 Mol) gasförmigem Schwefeltrioxid entsprechend einem molaren Einsatzverhältnis von 1 : 1,2 umgesetzt. Das SO₃ wurde unter Erwärmen aus einer entsprechenden Menge 65 gew.-%igen Oleums ausgetrieben, mit Stickstoff auf eine Konzentration von 5 Vol.-% verdünnt und innerhalb von 60 min in den Ester eingeleitet. Die Temperatur des Reaktionsgemisches wurde dabei unterhalb von 90°C gehalten.
Nach der Sulfonierung wurde das rohe Sulfonierungsprodukt zunächst 15 min lang einer Nachreaktion bei 80°C im Wasserbad unterworfen und anschließend durch Einrühren in eine 25 gew.-%ige wässrige Natriumhydroxidlösung neutralisiert.

Vor der Bestimmung der Klettfarbzahl wurde das alpha-Sulfofettsäureniedrigalkylester-Salz nach Zugabe von 2 Gew.-% Natriumhypochlorit, bezogen auf den Anteil an Waschaktivsubstanz, 120 min lang bei 60°C gebleicht.

Die Bestimmung der Klettfarbzahl der Produkte erfolgte mit einem Klettphotometer (Modell 800-3, Fa.Klett-Summerson, 1 cm Rundküvette, Blaufilter 400 - 465 nm) bei einer Waschaktivsubstanzkonzentration von 5 Gew.-% und pH = 7.

Die Gehalte an Oxoverbindungen und ungesättigten Verbindungen, die nach Vorbehandlung der Ester I - III erreicht wurden, sowie die Klettfarbzahlen der resultierenden Estersulfonate sind in Tab.3 (erfindungsgemäße Beispiele) und Tab.4 (Vergleichsbeispiele) zusammengefaßt. Die Ergebnisse stellen die Mittel von 5 Messungen dar.

**Tab.3**

| Gehalte an Oxoverbindungen und ungesättigten Verbindungen und Klettfarbzahlen (erfindungsgemäße Beispiele) | | | | | |
|---|---|---|---|---|---|
| Bsp. | Verfahren | Ester | Oxoverbindungen Gew.-% | unges. Verbindungen Gew.-% | Farbzahlen Klett |
| 1.1 | A | I | 0,07 | 0,09 | 23 |
| 1.2 | B | I | 0,07 | 0,05 | 12 |
| 1.3 | C | I | 0,06 | 0,05 | 10 |
| 1.4 | D | I | 0,06 | 0,05 | 9 |
| 2.1 | A | II | 0,09 | 0,09 | 29 |
| 2.2 | B | II | 0,09 | 0,05 | 19 |
| 2.3 | C | II | 0,08 | 0,05 | 14 |
| 2.4 | D | II | 0,08 | 0,05 | 12 |
| 3.1 | A | III | 0,06 | 0,09 | 17 |
| 3.2 | B | III | 0,06 | 0,05 | 10 |
| 3.3 | C | III | 0,05 | 0,05 | 8 |
| 3.4 | D | III | 0,05 | 0,05 | 6 |

**Tab.4**

| Gehalte an Oxoverbindungen und ungesättigten Verbindungen und Klettfarbzahlen (Vergleichsbeispiele) | | | | | |
|---|---|---|---|---|---|
| Bsp. | Verfahren | Ester | Oxoverbindungen Gew.-% | unges. Verbindungen Gew.-% | Farbzahlen Klett |
| V1.1 | E | I | 0,12 | 0,50 | 72 |
| V1.2 | F | I | 0,12 | 0,11 | 35 |
| V1.3 | G | I | 0,12 | 0,05 | 31 |
| V1.4 | H | I | 0,07 | 0,51 | 61 |
| V1.5 | I | I | 0,07 | 0,29 | 41 |
| V1.6 | J | I | 0,06 | 0,50 | 57 |
| V1.7 | K | I | 0,07 | 0,31 | 44 |
| V2.1 | E | II | 0,25 | 0,49 | 98 |
| V2.2 | F | II | 0,25 | 0,12 | 44 |
| V2.3 | G | II | 0,25 | 0,05 | 36 |
| V2.4 | H | II | 0,09 | 0,52 | 85 |
| V2.5 | I | II | 0,09 | 0,33 | 51 |
| V2.6 | J | II | 0,08 | 0,50 | 69 |
| V2.7 | K | II | 0,08 | 0,31 | 46 |

Die Bestimmung des Gehaltes an Oxoverbindungen und ungesättigten Verbindungen erfolgte gaschromatographisch.

## Patentansprüche

1. Verfahren zur Herstellung hellfarbiger waschaktiver alpha-Sulfettsäureniedrigalkylester-Salze durch Sulfonierung von Fettsäureniedrigalkylestern mit gasförmigem Schwefeltrioxid und nachfolgende Aufarbeitung, dadurch gekennzeichnet, daß man in die Sulfonierungsstufe Fettsäureniedrigalkylester einbringt,
a) deren Gehalt an Oxoverbindungen auf Werte kleiner 0,1 Gew.-% und
b) deren Gehalt an ungesättigten Verbindungen auf Werte kleiner 0,1 Gew.-%,
Gewichtsprozente jeweils bezogen auf Fettsäureniedrigalkylester, zuvor vermindert worden ist.

2. Verfahren nach Anspruch 1, dadurch gekennzeichnet, daß in die Sulfonierungsstufe Fettsäureniedrigalkylester eingesetzt werden, deren Gehalt an polaren Oxoverbindungen zuvor durch
a1) Destillation in Gegenwart von anorganischen Säuren oder deren Partialester mit Glycerin und/oder
a2) Adsorption an anorganischen Feststoffen
auf Werte kleiner 0,1, vorzugsweise kleiner 0,05 Gew.-%, bezogen auf Fettsäureniedrigalkylester, vermindert wurde.

3. Verfahren nach Anspruch 2, dadurch gekennzeichnet, daß anorganische Säuren in einer Konzentration von 0,05 bis 0,5 Gew.-%, bezogen auf den Fettsäureniedrigalkylester, in die Destillationsstufe eingesetzt werden.

4. Verfahren nach den Ansprüchen 2 und 3, dadurch gekennzeichnet, daß als anorganische Säuren Phosphorsäure, Unterphoshorige Säure und insbesondere Borsäure sowie deren Partialester mit Glycerin eingesetzt werden.

5. Verfahren nach Anspruch 2, dadurch gekennzeichnet, daß anorganische Feststoffe in einer Konzentration von 10 bis 150, vorzugsweise 25 bis 100 Gew.-%, bezogen auf den Fettsäureniedrigalkylester, in die Adsorptionsstufe eingesetzt werden.

6. Verfahren nach den Ansprüchen 2 und 5, dadurch gekennzeichnet, daß als anorganische Feststoffe Aluminiumoxid, Titandioxid, Magnesiumoxid und insbesondere Silicagel eingesetzt werden.

7. Verfahren nach den Ansprüchen 2, 5 und 6, dadurch gekennzeichnet, daß anorganische Feststoffe mit einer Korngröße von 0,03 bis 0,3 mm, vorzugsweise 0,06 bis 0,2 mm, in die Adsorptionsstufe eingesetzt werden.

8. Verfahren nach mindestens einem der Ansprüche 2, 5, 6 und 7, dadurch gekennzeichnet, daß in der Adsorptionsstufe der anorganischen Feststoff dem Fettsäureniedrigalkylester zugesetzt, das Gemisch unter Rühren in einer Schutzgasatmossphäre über einen Zeitraum von 0,5 bis 5 h auf 40 bis 80°C, vorzugsweise 50 bis 70°C erhitzt und der anorganischen Feststoff anschließend auf mechanischem Wege wieder abgetrennt wird.

9. Verfahren nach Anspruch 1, dadurch gekennzeichnet, daß in die Sulfonierungsstufe Fettsäureniedrigalkylester eingesetzt werden, deren Gehalt an ungesättigten Verbindungen durch Härtung in Gegenwart üblicher Hydrierkatalysatoren auf Werte kleiner 0,1, vorzugsweise kleiner 0,05 Gew.-%, bezogen auf den Fettsäureniedrigalkylester, vermindert wurde.

10. Verfahren nach Anspruch 9, dadurch gekennzeichnet, daß die Härtung in Gegenwart von 0,5 bis 2, vorzugsweise 0,8 bis 1,2 Gew.-%, bezogen auf den Fettsäureester, eines handelsüblichen Nickelkatalysators bei Temperaturen von 150 bis 220, vorzugsweise 170 bis 190°C und 5 bis 50, vorzugsweise 15 bis 25 bar durchgeführt wird.

11. Verfahren nach den Ansprüchen 1 bis 11, dadurch gekennzeichnet, daß in die Sulfonierungsstufe Ester von Fettsäuren mit 6 bis 22 Kohlenstoffatomen mit Alkoholen mit 1 bis 4 Kohlenstoffatomen eingesetzt werden.

12. Verfahren nach Anspruch 11, dadurch gekennzeichnet, daß in die Sulfonierungsstufe Methylester von Fettsäuren mit 8 bis 18 Kohlenstoffatomen eingesetzt werden.

13. Verfahren nach mindestens einem der Ansprüche 1 bis 12, dadurch gekennzeichnet, daß Fettsäureniedrigalkylester in die Sulfonierungsstufe eingesetzt werden, die durch Spaltung natürlicher Fette und Öle, Reinigung der resultierenden Fettsäuren nach dem Umnetzverfahren und nachfolgende Veresterung mit Methanol hergestellt werden.

## Claims

1. A process for the production of light-colored washing-active α-sulfofatty acid lower alkyl ester salts by sulfonation of fatty acid lower alkyl esters with gaseous sulfur trioxide and subsequent working up, characterized in that fatty acid lower alkyl esters
a) in which the content of oxo compounds has been reduced to values below 0.1% by weight and
b) in which the content of unsaturated compounds has been reduced to values below 0.1% by weight
(percentages by weight based on fatty acid lower alkyl ester)
are introduced into the sulfonation stage.

2. A process as claimed in claim 1, characterized in that fatty acid lower alkyl esters in which the content of oxo compounds has been reduced to values below 0.1% by weight and preferably to values below 0.05% by weight, based on the fatty acid lower alkyl ester, by
a1) distillation in the presence of inorganic acids or partial esters thereof with glycerol and/or
a2) adsorption to inorganic solids
are introduced into the sulfonation stage.

3. A process as claimed in claim 2, characterized in that inorganic acids are introduced into the distillation stage in a concentration of 0.05 to 0.5% by weight, based on fatty acid lower alkyl ester.

4. A process as claimed in claims 2 and 3, characterized in that phosphoric acid, hypophosphorous acid and, in particular, boric acid and also partial esters thereof with glycerol are used as the inorganic acids.

5. A process as claimed in claim 2, characterized in that inorganic solids are introduced into the adsorption stage in a concentration of 10 to 150% by weight and preferably in a concentration of 25 to 100% by weight, based on the fatty acid lower alkyl ester.

6. A process as claimed in claims 2 and 5, characterized in that aluminium oxide, titanium dioxide, magnesium oxide and, in particular, silica gel are used as the inorganic solids.

7. A process as claimed in claims 2, 5 and 6, characterized in that inorganic solids having a particle size of 0.03 to 0.3 mm and preferably 0.06 to 0.2 mm are introduced into the adsorption stage.

8. A process as claimed in at least one of claims 2, 5, 6 and 7, characterized in that, in the adsorption stage, inorganic solids are added to the fatty acid lower alkyl ester, the mixture is heated with stirring for 0.5 to 5 h to 40 to 80°C and preferably to 50 to 70°C in an inert gas atmosphere and the inorganic solids are then mechanically removed.

9. A process as claimed in claim 1, characterized in that fatty acid lower alkyl esters in which the content of unsaturated compounds has been reduced to values below 0.1% by weight and preferably below 0.05% by weight, based on the fatty acid lower alkyl ester, by hydrogenation in the presence of typical hydrogenation catalysts are introduced into the sulfonation stage.

10. A process as claimed in claim 9, characterized in that the hydrogenation reaction is carried out, for example, using 0.5 to 2% by weight and preferably 0.8 to 1.2% by weight, based on the fatty acid ester, of a commercially available nickel catalyst at temperatures of 150 to 220°C and preferably at temperatures of 170 to 190°C and under pressures of 5 to 50 bar and preferably 15 to 25 bar.

11. A process as claimed in claims 1 to 11, characterized in that esters of C₆₋₂₂ fatty acids with C₁₋₄ alcohols are used in the sulfonation stage.

12. A process as claimed in claim 11, characterized in that methyl esters of C₈₋₁₈ fatty acids are used in the sulfonation stage.

13. A process as claimed in at least one of claims 1 to 12, characterized in that fatty acid lower alkyl esters produced by hydrolysis of natural fats and oils, purification of the resulting fatty acids by the rolling-up process and subsequent esterification with methanol are used in the sulfonation stage.

## Revendications

1. Procédé de fabrication de sels d'alkylesters inférieurs d'acides gras alpha-sulfonés détergents-actifs de teinte claire par sulfonation d'alkylesters inférieurs d'acides gras avec de l'anhydride sulfurique gazeux et retraitement suivant, caractérisé en ce que l'on introduit dans la phase de sulfonation des alkylesters inférieurs d'acides gras,
a) dont la teneur en composés oxo a été réduite auparavant à des valeurs inférieures à 0,1 % en poids et,
b) dont la teneur en composés non saturés a été réduite auparavant à des valeurs inférieures à 0,1 % en poids,
pourcentages en poids rapportés respectivement à l'alkylester inférieur d'acides gras.

2. Procédé selon la revendication 1, caractérisé en ce que :
on utilise pour préparer des estersulfonates de couleur claire, on utilise dans la phase de sulfonation des alkylesters inférieurs d'acides gras, dont la teneur en composés oxo a été réduite auparavant par,
a₁) distillation en présence d'acides minéraux ou de leurs esters partiels avec de la glycérine et/ou
a₂) adsorption sur des matières solides minérales,
à des valeurs inférieures à 0,1, de préférence inférieures
à 0,05 % en poids, rapportées aux alkylesters inférieurs d'acides gras.

3. Procédé selon la revendication 2, caractérisé en ce que l'on met en jeu dans l'étape de distillation des acides minéraux en une concentration de 0,05 à 0,5 % en poids, par rapport aux alkylesters inférieurs d'acides gras.

4. Procédé selon les revendications 2 et 3, caractérisé en ce que l'on met en jeu comme acides minéraux de l'acide phosphorique, de l'acide hypophosphoreux et en particulier de l'acide borique ainsi que leurs esters partiaux avec du glycérol.

5. Procédé selon la revendication 2, caractérisé en ce que l'on met en jeu dans l'étape d'adsorption des matières solides minérales en une concentration de 10 à 150, de préférence de 25 à 100 % en poids, par rapport aux alkylesters inférieurs d'acides gras.

6. Procédé selon les revendications 2 et 5, caractérisé en ce que l'on met en oeuvre comme matières minérales solides de l'oxyde d'aluminium, du dioxyde de titane, de l'oxyde de magnésium et en particulier du gel de silice.

7. Procédé selon les revendications 2 et 5, caractérisé en ce que l'on met en oeuvre dans l'étape d'adsorption des matières minérales solides avec une taille de grains de 0,03 à 0,3 mm, de préférence de 0,06 à 0,2 mm.

8. Procédé selon au moins une des revendications 2, 5, 6 et 7, caractérisé en ce que dans l'étape d'adsorption on ajoute la matière solide minérale à l'alkylester inférieur d'acides gras, on chauffe le mélange sous agitation dans une atmosphère de gaz protecteur pendant une durée de 0,5 à 5 h. de 40 jusqu'à 80°C, de préférence de 50 à 70°C et on sépare à nouveau ensuite la matière solide minérale par une voie mécanique.

9. Procédé selon la revendication 1, caractérisé en ce que dans l'étape de sulfonation, on met en oeuvre des alkylesters inférieurs d'acides gras, dont la teneur en composés non saturés est diminuée par durcissement en présence de catalyseurs d'hydrogénation usuels à des valeurs inférieures à 0,1, de préférence inférieures à 0,05 % en poids, rapportées aux alkylesters inférieurs d'acides gras.

10. Procédé selon la revendication 9, caractérisé en ce que l'on effectue le durcissement en présence de 0,5 à 2, de préférence de 0,8 à 1,2 % en poids, par rapport aux esters d'acides gras, d'un catalyseur au nickel du commerce à des températures de 150 à 220, de préférence de 170 à 190°C et de 5 à 50, de préférence de 15 à 25 bars.

11. Procédé selon les revendications 1 à 11, caractérisé en ce que l'on met en oeuvre dans l'étape de sulfonation des esters d'acide gras ayant de 6 à 22 atomes de carbone avec des alcools ayant de 1 à 4 atomes de carbone.

12. Procédé selon la revendication 11, caractérisé en ce que l'on met en oeuvre dans l'étape de sulfonation des esters méthyliques d'acides gras ayant de 8 à 18 atomes de carbone.

13. Procédé selon au moins une des revendications 1 à 12, caractérisé en ce que l'on met en oeuvre dans l'étape de sulfonation des alkylesters inférieurs d'acides gras, qui sont produits par décomposition de graines et huiles, purification des acides gras résultants selon le procédé de modification du réseau et estérification successive avec du méthanol.
